(19) Europäisches Patentamt
European Patent Office
Office européen des brevets

(11) Veröffentlichungsnummer: **0 219 092 B1**

(12) **EUROPÄISCHE PATENTSCHRIFT**

(45) Veröffentlichungstag der Patentschrift: **04.10.89**

(51) Int. Cl.⁴: **C07C 15/44**, C07C 2/02 // B01J31/22, B01J31/14

(21) Anmeldenummer: **86114180.2**

(22) Anmeldetag: **14.10.86**

(54) **Verfahren zur Herstellubg von Alenylaromaten.**

(30) Priorität: **17.10.85 DE 3536929**

(43) Veröffentlichungstag der Anmeldung: **22.04.87 Patentblatt 87/17**

(45) Bekanntmachung des Hinweises auf die Patenterteilung: **04.10.89 Patentblatt 89/40**

(84) Benannte Vertragsstaaten: **BE DE FR GB IT NL**

(56) Entgegenhaltungen:
**DE-A- 1 792 571**
**DE-A- 2 211 745**
**DE-A- 2 234 922**
**DE-B- 1 618 870**

**AZERBAIDZHANSKII KHIMICHESKII Zhurnal, Nr. 2, 1978A.G. AZIZOV et al. "Kodimerisierung von vinylaromatischen Verbindungen mit Ethylen über einem katalytischen System aus NiX2+Triphenylphosphit+Triäthylaluminium+Halogenderivate von Bor" Seiten 3-8**

(73) Patentinhaber: **BASF Aktiengesellschaft, Carl-Bosch-Strasse 38, D-6700 Ludwigshafen(DE)**

(72) Erfinder: **Sperling, Karin, Dr., Waldstrasse 112, D-6706 Wachenheim(DE)**
Erfinder: **Fischer, Martin, Dr., Elbinger Weg 1, D-6700 Ludwigshafen(DE)**

**EP 0 219 092 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von Alkenylaromaten der Formel I

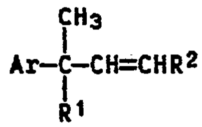

I,

worin $R^1$ einen Alkylrest mit 1 bis 6 Kohlenstoffatomen bedeutet, $R^2$ für Wasserstoff oder einen Alkylrest mit 1 bis 10 Kohlenstoffatomen steht und Ar einen substituierten oder unsubstituierten Arylrest darstellt, durch Umsetzung von Vinylaromaten der Formel II

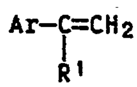

II.

mit einem α-Olefin der Formel III
$H_2C=CHR^2$ III
in Gegenwart eines nickelhaltigen Katalysatorsystems.

Es ist bekannt, daß mit einem Katalysatorsystem, bestehend aus $Ni(PCl_3)_4$, $AlBr_3$ und Butyllithium im molaren Verhältnis 0,25:5, 0:7,5 in Cyclohexan/-Hexan bei 15 bis 42°C Vinylaromaten mit Ethylen zu Alkylenaromaten verknüpft werden können (z.B. aus der DE-OS 1 792 571 oder C. Dixon, E.W. Duck, D.K. Jenkins, Organometal. Chem. Synth. 1 (1970/71) 1, 77–86). Nachteilig bei diesem Verfahren ist die aufwendige Synthese des Nickelkomplexes $Ni(PCl_3)_4$, der Einsatz von teurem Butyllithium im 30-fachen Überschuß für die Herstellung des Katalysators und die geringe Ausbeute von ca. 63%.

In der DE-B 1 618 870 wird ein Verfahren zur Dimerisierung, Codimerisierung, Polymerisierung und Copolymerisierung von Ethylen, Propylen und/oder Butylen mit einem Katalysatorsystem beschrieben, das neben dem Nickel(II)- oder Kobalt(II)salz als weitere Komponenten eine Lewis-Säure auf der Basis von Aluminium-, Beryllium-, Gallium-, und/oder Indium-alkylhalogeniden sowie eine Lewis-Base, biespielsweise Phospine oder Phosphite, enthält. Nachteilig an diesem Verfahren ist, daß bei der Codimerisierung von Ethylen mit Propylen und/oder Butylen der Anteil der dimeren und oligomeren Verbindungen im Produktgemisch den der gewünschten codimeren Verbindungen weit überwiegt. Die im Produktgemisch enthaltenen Dimeren und Codimeren liegen außerdem nicht als einheitliche Substanzen, sonder, aufgrund dere hohen Doppelbindungsisomerisierungsaktivität des angewandten Katalysatorsystems, als technisch nicht trennbare Isomerengemische vor. Die nach diesem Verfahren erhaltenen Produktgemische sind technisch nicht verwertbar.

Desweiteren ist die Umsetzung von Styrol und α-Methylstyrol mit Ethylen in Gegenwart von Nickeldiacetylacetonat, Triphenylphosphit, Triethylaluminium und $BF_3 \cdot Et_2O$ im molaren Verhältnis von 1:1:3:5 bei 25°C mit Ausbeuten von ca. 97% in Azerb. Khim. Zh. 1987, (2), 3–8 beschrieben. Dieses Verfahren besitzt jedoch den Nachteil, daß der Katalysator in Gegenwart einer fünften Komponente bei sehr tiefer Temperatur von –78°C Butadien erzeugt und die Verknüpfung der Olefine unter Druck im Autoklaven durchgeführt werden muß. Zur Erzielung guter Ergebnisse ist es nötig, die Bortrifluoridkomponente im hohen Überschuß zu verwenden.

Demgemäß war es Aufgabe der Erfindung, ein besseres nickelhaltiges Katalysatorsystem für die Umsetzung von Vinylaromaten mit α-Olefinen zu Alkenylaromaten, die wichtige Ausgangsstoffe für die Herstellung von z.B. Alkylaromaten und Anthrachinonderivaten sind, zur Verfügung zu stellen. Das Katalysatorsystem sollte möglichst einfach und billig zugänglich sein und unter milden Reaktionsbedingungen einen hohen Umsatz bei hoher Selektivität ermöglichen.

Es wurde nun gefunden, daß man Alkenylaromaten der Formel I

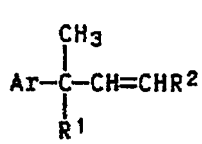

I,

worin $R^1$ einen Alkylrest mit 1 bis 6 Kohlenstoffatomen bedeutet, $R^2$ für Wasserstoff oder einen Alkylrest mit 1 bis 10 Kohlenstoffatomen steht und Ar einen substituierten oder unsubstituierten Arylrest darstellt, durch Umsetzung von Vinylaromaten der Formel II

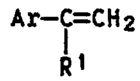 II,

mit einem α-Olefin der Formel III
$H_2C{=}CHR^2$ III
in Gegenwart eines nickelhaltigen Katalysatorsystems besonders vorteilhaft herstellt, wenn man ein Katalysatorsystem verwendet, welches durch Mischen von

a) einem einheitlichen oder gemischten Nickel(II)salz einer Carbonsäure, eines Alkohols oder eines Phenols oder Mischungen dieser Salze,
b) einer dreiwertigen organischen Phosphorverbindung der Formel IV

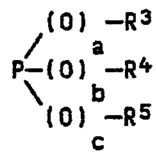 IV

in der die Reste $R^3$ bis $R^5$ für einen Alkyl-, Cycloalkyl-, Aryl- oder Aralkylrest stehen und die Indices a–c Null oder 1 bedeuten und
c) einer aluminiumorganischen Verbindung der Formel V

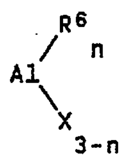 V

in der $R^6$ gleiche oder verschiedene Alkylgruppen mit 1 bis 12 Kohlenstoffatomen darstellt, X für Halogen steht und n die Zahl 1 oder 2 bedeuten oder Gemischen von zwei Verbindungen der Formel V hergestellt worden ist, wobei das Molverhältnis von Nickel zu Phosphor 1:0,5 bis 1:10 und von Nickel zu Aluminium 1:1 bis 1:20 beträgt.

Die Umsetzung wird bei Verwendung von α-Methylstyrol und Ethylen als Ausgangsstoffe durch folgende Reaktionsgleichung beschrieben:

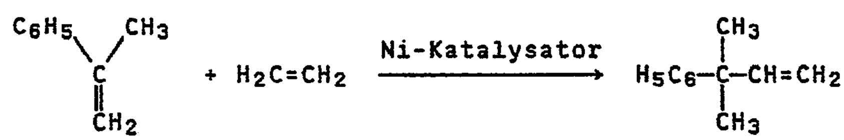


Als Ausgangsstoffe II kommen Vinylaromaten in Betracht, bei denen $R^1$ einen Alkylrest mit 1 bis 6, vorzugsweise 1 bis 4, insbesondere 1 und 2 Kohlenstoffatomen bedeutet. So steht der Rest $R^1$ beispielsweise für einen Methyl-, Ethyl-, n-Propyl, iso-Propyl-, n- oder iso-Butyl-, n- oder iso-Pentyl- oder Hexylrest. Besonders geeignet sind α-Methyl- und α-Ethylstyrole.

Der Arylrest steht z.B. für einen Phenyl- oder Naphtylrest, der noch unter den Reaktionsbedingungen inerte Substituenten wie Alkyl- oder Alkoxyreste mit 1 bis 6, insbesondere 1 bis 4 Kohlenstoffatomen wie Methyl, Ethyl, Propyl, Isopropyl, Butyl, Isobutyl und tertiär-Butyl und die entsprechenden Alkoxyreste oder Halogen wie Fluor, Chlor, Brom oder Jod tragen kann.

Als Ausgangsstoffe II seinen beispielsweise folgende Verbindungen genannt: α-Methylstyrol, α-Ethylstyrol, α-n-Propylstyrol, α-n-Butylstyrol, p-Methyl- und p-Methoxy-α-methylstyrol, p-Ethyl-, p-Isopropyl- und p-tertiär-Butyl-α-methylstyrol, p-Brom-, p-Chlor-α–methylstyrol und die entsprechenden o- und m-substituierten α–Methylstyrole, sowie p-Ethyl- oder p-Methyl-α-ethylstyrol und 1-Isopropylennaphtalin.

α-Olefine der Formel III sind solche, in denen $R^2$ Wasserstoff oder einen Alkylrest mit 1 bis 10, insbesondere 1 bis 6, vorzugsweise 1 bis 4 Kohlenstoffatomen bedeutet. Beispielsweise seien Ethen, Propen, But-1-en, Pent-1-en, Hex-1-en, Hept-1-en, Oct-1-en, Non-1-en, Dec-1-en, Neohexen, 3-Methyl-but-1-en, 4-Methyl-pent-1-en, 6-Ethyl-hept-1-en oder 4-tertiär-Butyl-hept-1-en genannt.

Die Ausgangsstoffe II werden mit den Ausgangsstoffen III im allgemeinen in stöchiometrischer Menge oder im Überschuß, vorzugsweise in einer Menge von 1 bis 2, insbesondere 1 bis 1,5 mol Ausgangsstoff III je Mol Ausgangsstoff II verwendet.

Das nickelhaltige Katalysatorsystem kann hergestellt werden, indem man ein Nickel-(II)-salz einer Carbonsäure, eines Alkoholates eines gesättigten aliphatischen Alkohols oder eines Phenolates mit a) einem Phosphit oder Phosphan und b) einer aluminiumorganischen Verbindung V mischt.

Nickel-(II)-salze von Alkoholaten bzw. Phenolaten sind beispielsweise $Ni(O{-}CH_3)_2$, $Ni(O{-}C_2H_5)_2$, $Ni(O{-}i{-}C_3H_7)_2$; $Ni(O{-}C_4H_9)_2$, $Ni(O\text{-tert.}{-}C_4H_9)_2$ und $Ni(O{-}C_6H_5)_2$. Die genannten organischen Gruppen können noch unter den Reaktionsbedingungen inerte Substituenten wie niedermolekulare Alkyl- oder Alkoxygruppen oder Halogen tragen. Besonders bevorzugt für die Herstellung des erfindungsgemäßen Katalysatorsystems sind Nickel-(II)-salze der Formel VI,

$Ni(OOCR^7)(OOCR^8)$ VI

in der $R^7$ und $R^8$ gleich oder verschieden sind und einen aliphatischen, araliphatischen oder aromatischen Rest, z.B. einen Alkyl-, Halogenalkyl-, Alkenyl-, Cycloalkyl-, Aryl- oder Alkenylrest darstellen. Die Kohlenstoffzahl der organischen Reste ist lediglich im Hinblick auf gute Löslichkeit der Nickelsalze im verwendeten Lösungsmittel von Bedeutung. So werden in unpolaren Lösungsmitteln vorzugsweise die Salze langkettiger Carbonsäuren, in denen die Summe $R^7$ und $R^8$ 5 bis 25 beträgt, verwendet. Bevorzugt können die in der europäischen Patentschrift EP 24 971 beschriebenen gemischten Nickelsalze eingesetzt werden, in denen $R^7$ für einen substituierten oder unsubstituierten Kohlenwasserstoffrest mit mindestens 5 Kohlenstoffatomen steht und $R^8$ einen Halogenalkylrest mit 1 bis 3 Kohlenstoffatomen, z.B. einen Trifluor- oder Trichlormethylrest bedeutet.

Beispielsweise können die Nickel-(II)-salze folgender Carbonsäuren einzeln oder im Gemisch miteinander verwendet werden: Essigsäure, Phenylessigsäure, Chloressigsäure, Trifluor-, Trichlor-, Tribrom- und Triiodessigsäure, Propionsäure, 2-Chlorpropionsäure, Buttersäure, Isobuttersäure, n-Valeriansäure, 4-Methylvaleriansäure, Capronsäure, 2-Ethylhexancarbonsäure, Önanthsäure, Caprylsäure, Caprinsäure, Laurinsäure, Myristinsäure, Palmitinsäure, Stearinsäure, Ölsäure, Cyclohexancarbonsäure, Benzoesäure, p-Chlorbenzoesäure, m-Toluylsäure. Die genannten Carbonsäuren können noch unter den Reaktionsbedingungen inerte Gruppene wie Alkyl- oder Alkoxygruppen mit 1 bis 4 Kohlenstoffatomen oder Halogen tragen. Es können auch Nickel-(II)-salze von Dicarbonsäuren als Katalysatorkomponente verwendet werden.

Als dreiwertige organische Phosphorverbindungen kommen tertiäre Phosphine oder tertiäre Phosphite der Formel IV in Betracht,

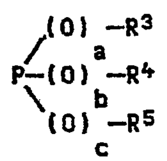

IV

in der die Reste $R^3$ bis $R^5$ gleich oder verschieden sind und für eine Alkylgruppe wie die Methyl-, Ethyl, Propyl-, Isopropyl-, Butyl-, tertiär-Butyl- oder Octylgruppe, eine Cycloalkylgruppe wie die Cyclohexylgruppe, eine Alkarylgruppe wie die Benzylgruppe oder eine Arylgruppe wie die Phenyl- oder Tolylgruppe stehen. Typische Beispiele derartiger Phosphorverbindungen sind: $P(CH_3)_3$, $P(C_2H_5)_3$, $P(n{-}C_3H_7)_3$, $P(n{-}C_4H_9)_3$, $P(\text{tert.}{-}C_4H_9)_3$, $P(C_8H_{17})_3$, $P(C_6H_{11})_3$, $P(C_6H_5)_3$, $P(CH_2C_6H_5)_3$, $P(C_2H_5)_2(C_6H_5)$, $P(C_2H_5)(C_6H_5)_2$, $P(OCH_3)_3$, $P(O{-}C_2H_5)_3$, $P(O{-}n{-}C_3H_7)_3$, $P(O{-}i{-}C_3H_7)_3$, $P(O{-}n{-}C_4H_9)_3$, $P(O{-}C_6H_5)_3$, $P(O{-}CH_2C_6H_5)_3$ und $P(OCH_3)(C_6H_5)_2$.

Als aluminiumorganische Verbindung V

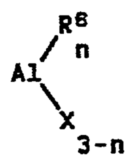

V

kommen insbesondere solche in Betracht, bei denen $R^6$ für gleiche oder verschiedene Alkylgruppen mit 1 bis 12, vorzugsweise 1 bis 6, insbesondere 1 bis 4 Kohlenstoffatome steht, z.B. für Methyl-, Ethyl-, n-Propyl-, iso-Propyl, n-Butyl- oder iso-Butylgruppen; X Fluor, Chlor, Brom oder Jod bedeutet und n die Zahl 1 oder 2 darstellt, oder Gemische von zwei verschiedenen Verbindungen der Formel V.

Beispielsweise seien folgende Verbindungen aufgeführt: Methylaluminiumdichlorid und -dibromid, Ethylaluminiumdichlorid und -dibromid, iso-Propylaluminiumdibromid, n-Butylaluminiumdichlorid, Dimethylaluminiumchlorid, Diethylaluminiumchlorid und -bromid. Gemische dieser Verbindungen sind z.B. Ethylaluminiumsesquichlorid. Bevorzugt können Gemische von Alkylaluminiumdichlorid in einem Molverhältnis von z.B. 20:1 bis 2:1, insbesondere 10:1 bis 5:1 verwendet werden.

Der Katalysator kann hergestellt werden, indem man vorzugsweise unter Inertgas wie Stickstoff oder Argon das Nickel(II)salz in einem Lösungsmittel mit der dreiwertigen organischen Phosphorverbindung und der aluminiumorganischen Verbindung bei einer Temperatur zwischen −20 und +80°C, insbesondere bei Raumtemperaturen mischt. Vorzugsweise kann man zunächst das Nickelsalz mit der Phosphorverbindung mischen und anschließend die aluminiumorganische Verbindung zugeben. Dabei erhält man eine Lösung des Katalysators, welche unmittelbar für die Umsetzung verwendet werden kann.

Als Lösungsmittel für die Katalysatorherstellung können vorteilhaft aromatische Kohlenwasserstoffe wie Toluol, Benzol, Ethylbenzol, o-, m-, p-Xylol oder aliphatische Kohlenwasserstoffe wie Pentan, Hexan, Heptan, Nonan, Petrolether oder cycloaliphatische Kohlenwasserstoffe wie Cyclohexan sowie halogenierte Kohlenwasserstoffe wie Chlorbenzol oder Methylenchlorid oder entsprechende Gemische verwendet werden.

Die Lösungsmittelmenge ist nicht kritisch. Es wird im allgemeinen soviel Lösungsmittel verwendet, daß der Katalysator darin gelöst ist und daß nach Zugabe der Reaktionspartner II und III gut rührbare Gemische vorliegen. Um die Menge des Lösungsmittels möglichst gering zu halten, kann es vorteilhaft sein, einen Teil des nach der Umsetzung anfallenden rohen Reaktionsgemisches als Reaktionsmedium für die Katalysatorherstellung und nachfolgende Umsetzung wiederzuverwenden.

Es ist nicht genau geklärt, auf welche Weise das Katalysatorsystem wirkt. Eine hohe katalytische Aktivität wird insbesondere dann festgestellt, wenn das Molverhältnis Nickel zu Phosphor 1:0,5 bis 1:10, vorzugsweise 1:1 bis 1:3, beträgt, und das Molverhältnis Nickel zu Aluminium bei 1:1 bis 1:20, bevorzugt bei 1:3 bis 1:10 liegt.

Es genügen im allgemeinen schon geringe Mengen des Katalysators, um bei der Umsetzung von II mit III hohen Umsatz bei hohen Selektivitäten zu gewährleisten. In der Regel beträgt die Menge des Katalysatorsystem 0,01 bis 0,0001, insbesondere 0,001 bis 0,0002 Mol.-% Nickel pro Mol II.

Das erfindungsgemäße Verfahren kann z.B. in der Weise durchgeführt werden, daß man zu dem Katalysatorsystem, bestehend aus dem Nickel(II)-salz VI, der organischen Phosphorverbindung IV und der aluminiumorganischen Verbindungen V in einem Lösungsmittel oder gelöst in Teilen des aus einer vorhergehenden Umsetzung erhaltenen Reaktionsgemisches, die Ausgangsstoffe II und III etwa gleichzeitig hinzugibt.

Sowohl die Herstellung des Katalysators als auch die katalytische Umsetzung werden bevorzugt in einer Inertgasatmosphäre unter weitgehendem Ausschluß von Wasser durchgeführt. Als Inertgas kommen z.B. Stickstoff, Argon oder Helium in Betracht. Die Reaktionstemperatur beträgt im allgemeinen 0 bis 80°C, insbesondere 30 bis 45°C. Die erfindungsgemäße Umsetzung kann kontinuierlich oder diskontinuierlich nach den dafür üblichen Techniken bei Normaldruck oder geringem Überdruck von etwa 1 bis 6 bar durchgeführt werden.

Die Aufarbeitung des rohen Reaktionsgemisches erfolgt in an sich bekannter Weise, indem man, ggf. nach Zersetzung des Katalysators z.B. mit wäßriger Lösung, die Reaktionsprodukte bzw. vorliegende Ausgangsstoffe destillativ aus dem Gemisch abtrennt.

Die nach dem erfindungsgemäßen Verfahren in hoher Ausbeute anfallenden Alkenylaromaten mit allylständiger Doppelbindung sind wertvolle, auf anderem Wege nur schwer zugängliche Zwischenprodukte für die Herstellung von Alkylaromaten, Alkylanthrachinonen, Farbstoffen, Riechstoffen oder Polymeren.

<u>Beispiel 1</u>

Unter Stickstoff wurden 50 ml trockenes Toluol, 1,2 ml (2 mmol) einer Lösung von Nickel(trifluoracetat)(etylhexanoat) in Toluol (1,7 molar) und 0,6 ml (2,3 mmol) Triphenylphosphit vorgelegt. Dann wurden bei Raumtemperatur 0,8 ml (10 mmol) einer 20,8%igen Lösung von Ethylaluminiumdichlorid in Hexan zugetropft. Zu dieser Katalysatorlösung wurden anschließend 472 g ( 4 mol) α-Methylstyrol und Ethylengas innerhalb vcn 4 h hinzugeben, wobei der Gasstrom so eingestellt wurde, daß kaum Abgas entwich. Die Ethylen-Aufnahme betrug ca. 20 l/h. die Temperatur wurde durch Wasserkühlung zwischen 35 und 40°C gehalten. Anschließend wurde das Reaktionsgemisch 1 h unter Einleitung von Ethylen bei der angegebenen Temperatur gerührt.

Zusammensetzung des Reaktionsgemisches:
3-Methyl-3-phenylbut-1-en: 83,3 Gew.-%
α-Methylstyrol: 3,6 Gew.-%

Als Nebenprodukte waren zu ca. 2% Butene und zu ca. 3% Co-trimere aus 1 Molekül α-Methylstyrol und 2 Molekülen Ethylen enthalten (z.B. 5-Methyl-5-phenylhex-2-en und Isomere). Der Umsatz an α-Methylstyrol betrug 95%, die Selektivität bezogen auf 3-Methyl-3-phenylbut-1-en 91%.

<u>Beispiel 2</u>

Die Durchführung erfolgte analog Beispiel 1, jedoch mit 50 g einer Reaktionsmischung, wie sie nach Beispiel 1 erhalten wurde, als Lösungsmittel und mit 1,2 ml (2 mmol) Nickel(trifluoracetat)(ethylhexanoat), 0,6 ml (2,3 mmol) Triphenylphosphit und 8,0 ml (12 mmol) Ethylaluminiumdichlorid (25%ig in Hexan) als Katalysatorsystem. 236 g (2 mol) α-Methylstyrol und Ethylengas wurden innerhalb von 3 h hinzugegeben und anschließend wurde 1 h unter Einleiten von Ethylen nachgerührt.

Zusammensetzung des Reaktionsgemisches:
3-Methyl-3-phenylbut-1-en: 87,7 Gew.-%
α-Methylstyrol: 5,25 Gew.-%

Umsatz: 92%
Selektivität: 97%

Beispiel 3

Die Durchführung erfolgte analog Beispiel 1, jedoch mit 50 g einer Reaktionsmischung, wie sie nach Beispiel 1 erhalten wurde, als Lösungsmittel und 1,21 ml (2,2 mmol) Nickel-di-(ethylhexanoat) (10%ig in Toluol), 0,66 ml (2,8 mmol) Triphenylphosphit und 8,8 ml (13,2 mmol) Ethylaluminiumdichlorid (25%ig in Hexan) als Katalysatorsystem. 236 g ( 2 mol) α-Methylstyrol und Ethylengas wurden innerhalb von 2 h hinzugegeben und anschließend wurde 1 h unter Einleiten von Ethylen nachgerührt.
Zusammensetzung des Reaktionsgemisches:
3-Methyl-3-phenylbut-1-en: 90,7 Gew.-%
α-Methylstyrol: 1,3 Gew.-%
Umsatz: 98%
Selektivität: 98%

Beispiel 4

Die Durchführung erfolgte analog Beispiel 1, jedoch wurde Propen anstelle von Ethylen mit 236 g (2 mol) α-Methylstyrol umgesetzt. Als Katalysatorlösung wurden 1,2 ml (2 mmol) Nickel(trifluoracetat)(ethylhexanoat), 0,6 ml (2,3 mmol) Triphenylphosphit und 18 ml (20 mmol) Ethylaluminiumdichlorid (in Hexan) in 400 ml trockenem Toluol verwendet.
Zusammensetzung des Reaktionsgemisches:
4-Methyl-4-phenylpent-1-en: 88 Gew.-%
α-Methylstyrol: 7,8 Gew.-%
Umsatz: 92,5%
Selektivität: 91%

Beispiel 5

Die Durchführung erfolgte analog Beispiel 1, jedoch mit 50 ml abs. $CH_2Cl_2$ als Lösungsmittel, 1,32 ml (2,75 mmol) Nickel(trifluoracetat)(ethylhexanoat), 0,55 ml (2,8 mmol) Triphenylphosphit und 10,3 ml (15,4 mmol) Ethylaluminiumdichlorid (25%ig in Hexan).
236 g (2 mol) α-Methylstyrol und Ethylengas wurden innerhalb von 3 h hinzugegeben und anschließend wurde 1 h unter Einleiten von Ethylen nach gerührt.
Zusammensetzung des Reaktionsgemisches:
3-Methyl-3-phenyl-but-1-en: 53 Gew.-%
α-Methylstyrol: 0,98 Gew.-%
Umsatz: 98%
Selektivität: 59%

Beispiel 6

Die Durchführung erfolgte analog Beispiel 1 in 50 ml Toluol, jedoch mit 0,55 ml (1 mmol) Nickel-di-(ethylhexanoat) (10%ig in Toluol), 0,3 ml (1,3 mmol) Triphenylphosphit und 4,77 ml (6,6 mmol) einer 10:1 Mischung von $EtAlCl_2$ und $Et_2AlCl$ in Hexan als Katalysatorsystem. 236 g (2 mol) α-Methylstyrol und Ethylengas wurden innerhalb von 2 h hinzugegeben und anschließend 1 h unter Einleiten von Ethylen nachgerührt.
Zusammensetzung des Reaktionsgemisches:
3-Methyl-3-phenyl-but-1-en: 86,3 Gew.-%
α-Methylstyrol: 7,4 Gew.-%
Umsatz: 90%
Selektivität: 97%

Beispiel 7

Die Durchführung erfolgte analog Beispiel 1 in 50 ml Toluol, jedoch mit 0,24 g (0,44 mmol) Nickel-di-(ethylhexanoat) (10%ig in Toluol), 0,15 g (0,57 mmol) Triphenylphosphan, 2 ml (2 mmol) einer 25%igen Lösung von $EtAlCl_2$ in Hexan als Katalysatorsystem.
236 g (2 mol) α-Methylstyrol und Ethylengas wurden innerhalb von 2 h hinzugegeben und anschließend wurde noch ca. 1 h unter Einleiten von Ethylen nachgerührt.
Zusammensetzung des Reaktionsgemisches:
3-Methyl-3-phenyl-but-1-en: 39 Gew.-%
α-Methylstyrol: 30,75 Gew.-%
Umsatz: 54%

Selektivität: 87%

Beispiel 8

Die Durchführung erfolgte analog Beispiel 1 in 50 ml abs. Toluol, jedoch mit 0,29 g (0,5 mmol) Nickel-di-(ethylhexanoat), 0,40 g (1,3 mmol) Triphenylphosphit, 4,2 ml (6 mmol) $EtAlCl_2$ (25%ig in Hexan) und 1,2 ml (1,2 mmol) $Et_2AlCl$ (1 molar in Hexan) als Katalysatorsystem.

264 g (2 mol p-Methyl-α-methylstyrol wurden in 4 h unter Einleiten von Ethylen zugetropft und dann wurde 1 h unter Ethylengaszufuhr nachgeführt.

Zusammensetzung des Reaktionsgemisches:

3-Methyl-3-(p-methylphenyl)-but-1-en: 84,4 Gew.-%

p-Methyl-α-Methylstyrol:

Umsatz: 100%

Selektivität: 91%

Die Zusammensetzung des Reaktionsgemisches wurde in allen Beispielen gaschromatographisich ermittelt.

**Patentansprüche**

1. Verfahren zur Herstellung von Alkenylaromaten der Formel I

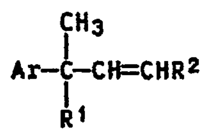

I,

worin $R^1$ einen Alkylrest mit 1 bis 6 Kohlenstoffatomen bedeutet, $R^2$ für Wasserstoff oder einen Alkylrest mit 1 bis 10 Kohlenstoffatomen steht und Ar einen substituierten oder unsubstituierten Arylrest darstellt, durch Umsetzung von Vinylaromaten der Formel II

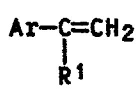

II,

mit einem α-Olefin der Formel III

$H_2C=CHR^2$ III

in Gegenwart eines nickelhaltigen Katalysatorsystems, dadurch gekennzeichnet, daß man ein Katalysatorsystem verwendet, welches durch Mischen von

a) einem einheitlichen oder gemischten Nickel(II)salz einer Carbonsäure, eines Alkohols oder eines Phenols oder Mischungen dieser Salze,

b) einer dreiwertigen organischen Phosphorverbindung der Formel IV

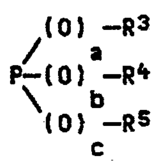

IV

in der Reste $R^3$ bis $R^5$ für einen Alkyl-, Cycloalkyl-, Aryl- oder Aralkylreste stehen und die Indices a–c Null oder 1 bedeuten und

d) einer aluminiumorganischen Verbindung der Formel V

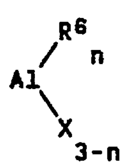

V

in der $R^6$ gleiche oder verschiedene Alkylgruppen mit 1 bis 12 Kohlenstoffatomen darstellt, X für Halogen steht und n die Zahl 1 oder 2 bedeutet oder Gemischen von zwei Verbindungen der Formel V hergestellt worden ist, wobei das Molverhältnis von Nickel zu Phosphor 1:0,5 bis 1:10 und von Nickel zu Aluminium 1:1 bis 1:20 beträgt.

2. Verfahren nach Anspruch 1, dadurch gekennzeichnet, daß man ein Nickel-(II)-salz der allgemeinen Formel VI

$Ni(OOCR^7)(OOCR^8)$ VI

in der $R^7$ und $R^8$ gleich oder verschieden sind und einen Alkyl-, Halogenalkyl-, Cycloalkyl-, Alkenyl-, Aryl-, Aralkyl- oder Alkarylrest darstellen, verwendet.

3. Verfahren nach Anspruch 2, dadurch gekennzeichnet, daß $R^7$ einen Kohlenwasserstoffrest mit 5 bis 20 Kohlenstoffatomen und $R^8$ einen durch Halogen substituierten Alkylrest mit 1 bis 3 Kohlenstoffatomen bedeuten.

4. Verfahren nach den Ansprüchen 1 bis 3, dadurch gekennzeichnet, daß man als phophororganische Verbindung ein Phosphit verwendet.

5. Verfahren nach den Ansprüchen 1 bis 4, dadurch gekennzeichnet, daß die Menge des Katalysatorsystems 0,01 bis 0,0001 Mol.-% Nickel pro Mol II beträgt.

## Claims

1. A process for the preparation of an alkenylaromatic of the formula I

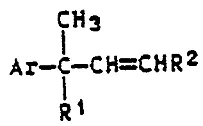

I

where $R^1$ is alkyl of 1 to 6 carbon atoms, $R^2$ is hydrogen or alkyl of 1 to 10 carbon atoms and Ar is unsubstituted or substituted aryl, by reacting a vinylaromatic of the formula II

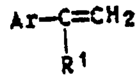

II

with an $\alpha$-Olefin of the formula III

$H_2C{=}CHR^2$ III

in the presence of a nickel-containing catalyst system, wherein the catalyst system used in prepared by mixing

a) a pure or mixed Nickel (II) salt of a carboxylic acid, of an alcohol or of a phenol or a mixture thereof,

b) a trivalent organic phosphorus compound of the formula IV

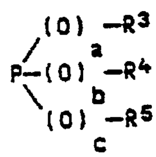

IV

where $R^3$, $R^4$ and $R^5$ are each alkyl, cycloalkyl, aryl or aralkyl and the indices a, b and c are zero or 1,

c) an organoaluminum compound of the formula V

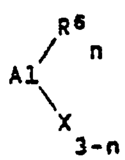

V

where the radicals $R^6$ are identical or different alkyl groups of 1 to 12 carbon atoms, X is halogen and n is 1 or 2, or a mixture of two compounds of the formula V,

the molar ratio of nickel to phosphorus being from 1:0.5 to 1:10 and that of nickel to aluminum being from 1:1 to 1:20.

2. A process as claimed in claim 1, wherein a nickel(II) salt of the formula VI

$Ni(OOCR^7)(OOCR^8)$ VI

where $R^7$ and $R^8$ are identical or different and are each alkyl, haloalkyl, cycloalkyl, alkenyl, aryl, aralkyl or alkaryl, is used.

3. A process as claimed in claim 2, wherein $R^7$ is a hydrocarbon radical of 5 to 20 carbon atoms and $R^8$ is a halogen-substituted alkyl radical of 1 to 3 carbon atoms.

4. A process as claimed in any of claims 1 to 3, wherein a phosphite is used as the organophosphorus compound.

5. A process as claimed in any of claims 1 to 4, wherein the amount of the catalyst system corresponds to 0.01–0.0001 mol.-% of nickel per mole of II.

**Revendications**

1. Procédé de préparation de carbures alcénylaromatiques de formule I

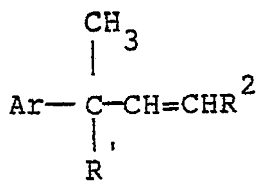

I,

où $R^1$ représente un rest alkyle ayant 1 à 6 atomes de carbone, $R^2$ est mis pour hydrogène ou un reste alkyl ayant 1 à 10 atomes de carbone et Ar représente un reste aryle substitué ou non substitué, par réaction de carbures vinylaromatiques de formule II

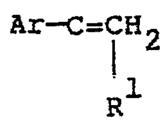

II,

avec un α-oléfine de formule III
$H_2C{=}CHR^2$ III,
en présence d'un système catalytique à teneur en nickel, caractérisé par le fait que l'on utilise un système catalytique qui a été préparé par mélange de

a) un sel de nickel (II), unique ou mélangé, d'un acide carboxylique, d'un alcool, ou d'un phénol, ou d'un mélange de ces sels,

b) un dérivé de phosphore organique, trivalent, de formule IV,

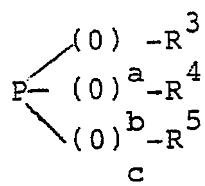

IV,

dans laquelle les restes $R^3$ à $R^5$ sont mis pour un reste alkyle, cycloalkyle, aryle ou aralkyle et les indices a–c représentent zéro ou 1, et

c) un composé organique d'aluminium de formule V

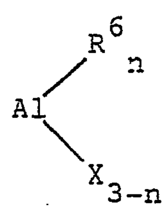

V,

dans laquel $R^6$ représente des groupes alkyle identiques ou différents ayant 1 à 12 atomes de carbone, X est mis pour halogène, et n représente le nombre 1 ou 2, ou des mélanges de deux composés de formule V,

le rapport molaire du nickel au phosphore étant de 1/0,5 à 1/10 et du nickel à l'aluminium étant de 1/1 à 1/20.

2. Procédé selon la revendication 1, caractérisé par le fait que l'on utilise un sel de nickel (II) de formule générale VI
$Ni(OOCR^7)(OOCR^8)$ VI,
dans laquelle $R^7$ et $R^8$ sont identiques ou différents et représentent un reste alkyle, halogénoalkyle, cycloalkyle, alcanyle, aryle, aralkyle ou alkaryle.

3. Procédé selon la revendication 2, caractérisé par le fait que $R^7$ représente un reste d'hydrocarbure ayant 5 à 20 atomes de carbone et $R^8$ un reste substitué par halogène et ayant 1 à 3 atomes de carbone.

4. Procédé selon les revendications 1 à 3, caractérisé par le fait que l'on utilise un phosphite, comme composé organique de phosphore.

5. Procédé selon les revendications 1 à 4, caractérisé par le fait que la quantité de système catalytique est de 0,01 à 0,0001 mole% de nickel par mole de II.